(19) Europäisches Patentamt
European Patent Office
Office européen des brevets

(11) Veröffentlichungsnummer: **0 318 796 B1**

# (12) EUROPÄISCHE PATENTSCHRIFT

(45) Veröffentlichungstag der Patentschrift: **16.06.93**

(51) Int. Cl.$^5$: **C07C 275/54**, C07C 335/26, C07C 331/28, C07C 265/12, A01N 47/34

(21) Anmeldenummer: **88119383.3**

(22) Anmeldetag: **22.11.88**

Die Akte enthält technische Angaben, die nach dem Eingang der Anmeldung eingereicht wurden und die nicht in dieser Patentschrift enthalten sind.

## (54) Substituierte Benzoyl(thio)harnstoffe.

(30) Priorität: **02.12.87 DE 3740807**

(43) Veröffentlichungstag der Anmeldung:
**07.06.89 Patentblatt 89/23**

(45) Bekanntmachung des Hinweises auf die Patenterteilung:
**16.06.93 Patentblatt 93/24**

(84) Benannte Vertragsstaaten:
**BE CH DE FR GB IT LI NL**

(56) Entgegenhaltungen:
**EP-A- 0 246 061**
**EP-A- 0 268 943**
**EP-A- 0 268 952**

(73) Patentinhaber: **BAYER AG**
**W-5090 Leverkusen 1 Bayerwerk(DE)**

(72) Erfinder: **Sirrenberg, Wilhelm, Dr.**
**Wuppertaler Strasse 21**
**W-4322 Sprockhövel(DE)**
Erfinder: **Marhold, Albrecht, Dr.**
**Carl-Duisberg-Strasse 329**
**W-5090 Leverkusen 1(DE)**
Erfinder: **Becker, Benedikt, Dr.**
**Metzkausener Strasse 14**
**W-4020 Mettmann(DE)**
Erfinder: **Stendel, Wilhelm, Dr.**
**In den Birken 55**
**W-5600 Wuppertal 1(DE)**

EP 0 318 796 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Benzoyl(thio)harnstoffe, Verfahren und neue Zwischenprodukte zu ihrer Herstellung sowie ihre Verwendung als Schädlingsbekämpfungsmittel, insbesondere als Insektizide.

Es ist bereits bekannt, daß bestimmte Benzoylharnstoffe, wie z. B. 1-(2-Chlor-benzoyl)-3-(4-chlor-3-trifiuormethyl-phenyl)-harnstoff, 1-(2,6-Difluor-benzoyl)-3-(4-fluor-3-trifluormethyl-phenyl)-harnstoff, 1-(2,6-Dichlor-benzoyl)-3-(4-fluor-3-trifluormethyl-phenyl)-harnstoff, 1-(2,6-Difluorbenzoyl)-3-(4-trifluormethyl-phenyl)-harnstoff und 1-(2,6-Difluor-benzoyl)-3-(3,5-Dichlor-2,4-difluor-phenyl)-harnstoff, insektizide Eigenschaften aufweisen (vgl. DE-OS 25 31 279 DE-OS 21 23 236 und EP-A 52 833). Weiterhin sind 1-(2,6-Difluor-benzoyl)-3-(2,5-difluor-4-trifluor-methyl-phenyl)-harnstoff und 1-(2,4,6-Trifluor-benzoyl)-3-(2,5-difluor-4-trifluor-methyl-phenyl)-harnstoff als insektizide Wirkstoffe bekannt geworden (vgl. EP-A 0 246 061).

Es wurden nun die neuen substituierten Benzoyl(thio)harnstoffe der allgemeinen Formel (I),

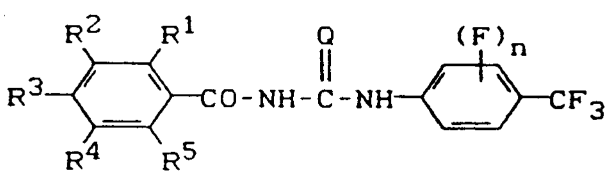

(I)

in welcher

- Q für Sauerstoff oder Schwefel steht,
- $R^1$ für Wasserstoff, Halogen, Nitro, Methyl oder Trifluormethyl steht,
- $R^2$ für Wasserstoff, Fluor oder Chlor steht,
- $R^3$ für Wasserstoff, Fluor oder Chlor steht,
- $R^4$ für Wasserstoff oder Fluor steht,
- $R^5$ für Wasserstoff oder Halogen steht und
- n für die Zahlen 2, 3 oder 4 steht,

ausgenommen die Verbindungen, in welchen zugleich

- $R^1$ und $R^5$ für Fluor stehen und
- $R^2$ und $R^4$ für Wasserstoff stehen und
- $R^3$ für Wasserstoff oder Fluor steht und
- Q für Sauerstoff steht und
- $(F)_n$ für 2,5-Difluor steht.

Weiter wurde gefunden, daß man diese neuen substituierten Benzoyl(thio)harnstoffe der allgemeinen Formel (I) erhält, wenn man

(a) substituierte Aniline der allgemeinen Formel (II),

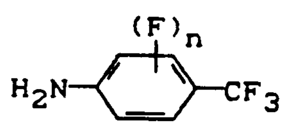

(II)

in welcher

n die oben angegebene Bedeutung hat,

mit Benzoyliso(thio)cyanaten der allgemeinen Formel (III)

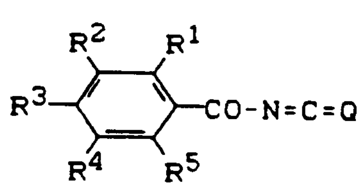

(III)

in welcher

Q, $R^1$, $R^2$, $R^3$, $R^4$ und $R^5$ die oben angegebenen Bedeutungen haben,

gegebenenfalls in Gegenwart eines Verdünnungsmittels umsetzt, oder

(b) substituierte Phenyliso(thio)cyanate der allgemeinen Formel (IV)

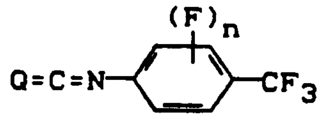

(IV)

in welcher

Q und n die oben angegebenen Bedeutungen haben,

mit Benzoesäureamiden der allgemeinen Formel (V)

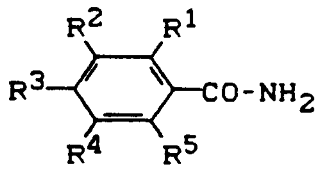

(V)

in welcher

$R^1$, $R^2$, $R^3$, $R^4$ und $R^5$ die oben angegebenen Bedeutungen haben,

gegebenenfalls in Gegenwart eines Katalysators und gegebenenfalls in Gegenwart eines Verdünnungsmittels umsetzt.

Die neuen Verbindungen der allgemeinen Formel (I) verfügen über überraschende, vorteilhafte Eigenschaften, die ihre Verwendung als Schädlingsbekämpfungsmittel ermöglichen; sie zeigen gute arthropodizide Eigenschaften und zeichnen sich insbesondere durch sehr starke insektizide Wirksamkeit aus.

In den allgemeinen Formeln bedeutet Halogen Fluor, Chlor, Brom und Iod, vorzugsweise Fluor, Chlor und Brom, besonders bevorzugt Fluor oder Chlor.

n steht vorzugsweise für 2 oder 3, wobei die Fluoratome bevorzugt in den 3,5- und 3,5,6-Stellungen stehen.

Bevorzugt werden die neuen substituierten Benzoyl(thio)harnstoffe der Formel (I), in welcher

Q für Sauerstoff oder Schwefel steht,

$R^1$ für Wasserstoff, Fluor, Chlor, Nitro, Methyl oder Trifluormethyl (vorzugsweise für Fluor, Chlor oder Methyl) steht,

$R^2$ für Wasserstoff, Fluor oder Chlor steht,

$R^3$ für Wasserstoff, Fluor oder Chlor steht,

$R^4$ für Wasserstoff oder Fluor steht,

$R^5$ für Wasserstoff, Fluor oder Chlor steht und

n für die Zahlen 2, 3 oder 4 steht.

Besonders bevorzugt werden die neuen substituierten Benzoyl(thio)harnstoffe der Formel (I), in welcher

Q für Sauerstoff oder Schwefel (vorzugsweise Sauerstoff) steht,

$R^1$ für Fluor oder Chlor steht,

$R^2$ für Wasserstoff steht,

$R^3$ für Wasserstoff oder Fluor steht,

$R^4$ für Wasserstoff oder Fluor (vorzugsweise Wasserstoff) steht,

$R^5$ für Wasserstoff oder Fluor steht und

n für die Zahlen 2, 3 oder 4 (vorzugsweise 2 oder 3) steht.

Beispiele für Verbindungen der Formel (I) sind in der nachstehenden Tabelle 1 aufgeführt - vgl. auch die Herstellungsbeispiele.

<u>Tabelle 1</u>: Beispiele für die Verbindungen der Formel (I)

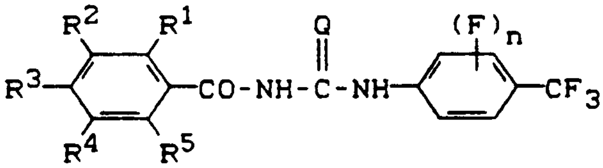 (I)

| Q | $R^1$ | $R^2$ | $R^3$ | $R^4$ | $R^5$ | 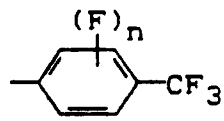 |
|---|---|---|---|---|---|---|
| O | F | H | H | H | F | 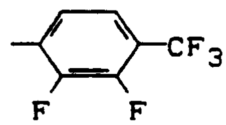 |
| S | F | H | H | H | F | 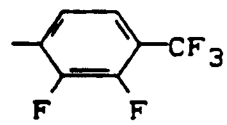 |
| O | Cl | H | H | H | H | 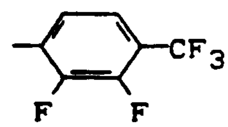 |

Tabelle 1 - Fortsetzung

| Q | $R^1$ | $R^2$ | $R^3$ | $R^4$ | $R^5$ | 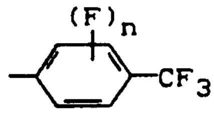 |
|---|---|---|---|---|---|---|
| S | Cl | H | H | H | H | 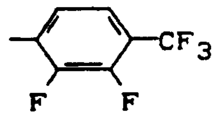 |
| O | Cl | H | H | H | F | 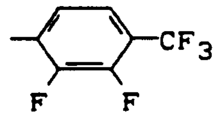 |
| S | Cl | H | H | H | F | 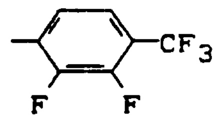 |
| O | Cl | H | F | H | H | 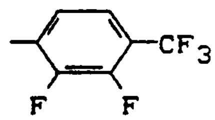 |
| S | Cl | H | F | H | H | 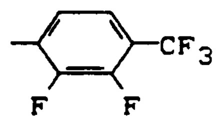 |
| O | Cl | H | H | F | H | 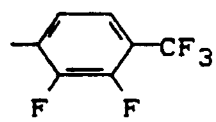 |
| S | Cl | H | H | F | H | 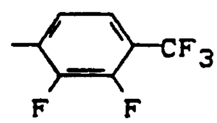 |
| O | F | H | H | H | H | 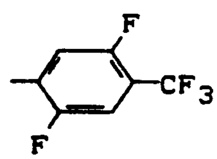 |

Tabelle 1 - Fortsetzung

| Q | $R^1$ | $R^2$ | $R^3$ | $R^4$ | $R^5$ | 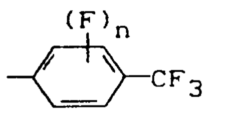 |
|---|---|---|---|---|---|---|
| S | F | H | H | H | H | 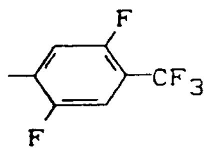 |
| O | Br | H | H | H | H | 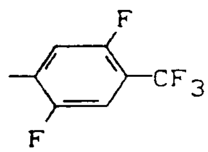 |
| S | Br | H | H | H | H | 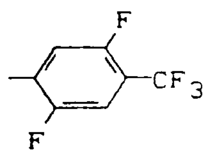 |
| O | Cl | H | H | H | H | 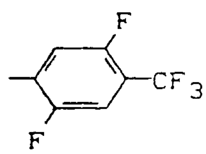 |
| S | Cl | H | H | H | H | 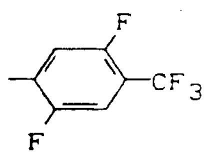 |
| O | Cl | H | H | H | F | 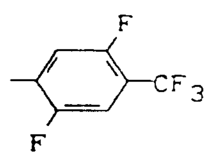 |

Verwendet man für die Verfahrensvariante (a) beispielsweise 2,5-Difluor-4-trifluormethyl-anilin und 2-Fluorbenzoylisothiocyanat als Ausgangsstoffe, so kann der Reaktionsablauf durch das folgende Formelschema wiedergegeben werden:

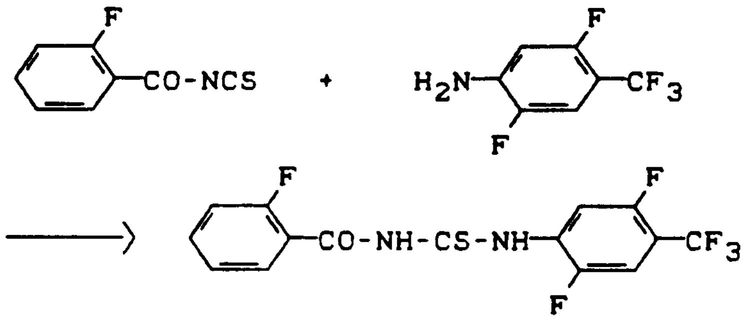

Verwendet man für die Verfahrensvariante (b) beispielsweise 2,3-Difluor-4-trifluormethyl-phenylisocyanat und 2,4-Difluor-benzoesäureamid als Ausgangsstoffe, so kann der Reaktionsablauf durch das folgende Formelschema wiedergegeben werden:

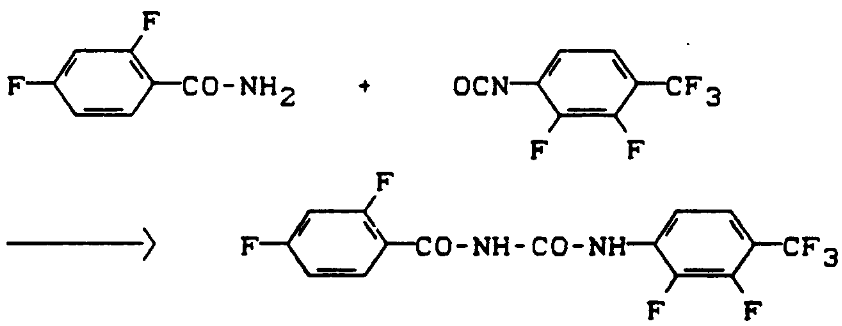

Die beim erfindungsgemäßen Verfahren (a) als Ausgangsstoffe zu verwendenden substituierten Aniline sind durch die Formel (II) allgemein definiert. In Formel (II) hat n vorzugsweise bzw. insbesondere die Bedeutung, die bereits im Zusammenhang mit der Beschreibung der erfindungsgemäßen Verbindungen der Formel (I) als bevorzugt bzw. als besonders bevorzugt angegeben wurde.

Als Beispiele für die Ausgangsstoffe der Formel (II) seien genannt:
2,3-Difluor-4-trifluormethyl-anilin, 2,5-Difluor-4-trifluormethyl-anilin, 2,6-Difluor-4-trifluormethyl-anilin, 3,5-Difluor-4-trifluormethyl-anilin, 2,3,5-Trifluor-4-trifluormethyl-anilin, 2,3,6-Trifluor-4-trifluormethyl-anilin und 2,3,5,6-Tetrafluor-4-trifluormethyl-anilin.

Die Ausgangsstoffe der Formel (II) sind neu.

Man erhält die Verbindungen der Formel (II), wenn man Benzotrifluorid-Derivate der allgemeinen Formel (VI)

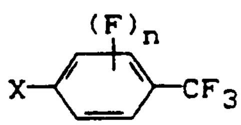

(VI)

in welcher

n die oben angegebene Bedeutung hat und

X für Fluor oder Chlor steht,

mit Ammoniak bei erhöhtem Druck bis etwa 20 bar und bei Temperaturen zwischen 10 °C und 150 °C, gegebenenfalls in Gegenwart eines Verdünnungsmittels, wie z. B. Tetrahydrofuran oder Dioxan, umsetzt und das Produkt der Formel (II) durch Destillation unter vermindertem Druck isoliert.

Die bei Verfahren (a) weiter als Ausgangsstoffe zu verwendenden Benzoyliso(thio)cyanate sind durch die Formel (III) allgemein definiert. In Formel (III) haben Q, $R^1$, $R^2$, $R^3$, $R^4$ und $R^5$ vorzugsweise bzw. insbesondere diejenigen Bedeutungen, die bereits im Zusammenhang mit der Beschreibung der erfindungsgemäßen Verbindungen der Formel (I) als bevorzugt bzw. als besonders bevorzugt für Q, $R^1$, $R^2$, $R^3$, $R^4$ und $R^5$ angegeben wurden.

Als Beispiele für die Ausgangsstoffe der Formel (III) seien genannt:
2-Fluor-, 2-Chlor-, 2-Brom-, 2-Iod-, 2,6-Difluor-, 2,4-Difluor-, 2-Chlor-4-fluor, 2-Chlor-6-fluor-, 2-Chlor-5-fluor-, 2,3,6-Trichlor-, 2,3,4,5,6-Pentafluor-, 2,4-Dichlor-5-fluor-, 2-Methyl-, 2-Nitro-, 2-Trifluormethyl-, 2,6-Dichlor-, 2,4-Dichlor- und 4-Chlor-benzoylisocyanat bzw. -benzoylisothiocyanat.

Die Ausgangstoffe der Formel (III) sind bekannt und/oder können nach an sich bekannten Verfahren hergestellt werden (vgl. DE-OS 21 23 236, DE-OS 25 04 982, DE-OS 26 01 780, DE-OS 28 01 316, DE-OS 28 37 086, DE-OS 32 17 619, DE-OS 32 17 620).

Die beim erfindungsgemäßen Verfahren (b) als Ausgangsstoffe zu verwendenden substituierten Phenyliso(thio)cyanante sind durch die Formel (IV) allgemein definiert. In Formel (IV) haben Q und n vorzugsweise bzw. insbesondere diejenigen Bedeutungen, die bereits im Zusammenhang mit der Beschreibung der erfindungsgemäßen Verbindungen der Formel (I) als bevorzugt bzw. als besonders bevorzugt angegeben wurden.

Als Beispiele für die Ausgangsstoffe der Formel (IV) seien genannt:
2,3-Difluor-4-trifluormethyl-, 2,5-Difluor-4-trifluormethyl-, 2,6-Difluor-4-trifluormethyl-, 3,5-Difluor-4-trifluormethyl-, 2,3,5-Trifluor-4-trifluormethyl-, 2,3,6-Trifluor-4-trifluormethyl- und 2,3,5,6-Tetrafluor-4-trifluormethyl-phenylisocyanat bzw. -phenylisothiocyanat.

Die Ausgangsstoffe der Formel (IV), ausgenommen die Verbindungen, in welchen zugleich $(F)_n$ für 2,5-Difluor steht und Q für Sauerstoff steht, sind neu und Teil der vorliegenden Erfindung.

Man erhält die Verbindungen der Formel (IV), wenn man entsprechende substituierte Aniline der allgemeinen Formel (II) in üblicher Weise mit Phosgen bzw. Thiophosgen, gegebenenfalls in Gegenwart eines Verdünnungsmitttels, wie z. B. Toluol, Chlorbenzol, Xylol oder o-Dichlorbenzol, bei Temperaturen zwischen 0 °C und 150 °C umsetzt und das Reaktionsprodukt anschließend unter vermindertem Druck destilliert.

Die bei Verfahren (b) weiter als Ausgangsstoffe zu verwendenden Benzoesäureamide sind durch die Formel (V) allgemein definiert. In Formel (V) haben $R^1$, $R^2$, $R^3$, $R^4$ und $R^5$ vorzugsweise bzw. insbesondere diejenigen Bedeutungen, die bereits im Zusammenhang mit der Beschreibung der erfindungsgemäßen Verbindungen der Formel (I) als bevorzugt bzw. als besonders bevorzugt angegeben wurden.

Als Beispiele für die Ausgangsstoffe der Formel (V) seien genannt:
2-Fluor-, 2-Chlor-, 2-Brom-, 2-Iod-, 2,6-Difluor-, 2,4-Difluor-, 2-Chlor-6-fluor-, 2-Chlor-4-fluor-, 2-Chlor-5-fluor-, 2,3,6-Trichlor-, 2,3,4,5,6-Pentafluor-, 2,4-Dichlor-5-fluor-, 2-Methyl-, 2-Nitro-, 2-Trifluormethyl-, 2,6-Dichlor-, 2,4-Dichlor- und 4-Chlor-benzoesäureamid.

Die Ausgangsstoffe der Formel (V) sind bekannt und/oder können nach an sich bekannten Verfahren hergestellt werden (vgl. Literatur zu den Ausgangsstoffen der Formel (III)).

Als Verdünnungsmittel kommen bei der Durchführung der Verfahrensvarianten (a) und (b) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z. B. Acetonitril und Propionitril, Amide, wie z. B. Dimethylformamid, Dimethylacetamid und N-Methyl-pyrrolidon, sowie Tetramethylensulfon.

Als Katalysatoren können für die Umsetzung gemäß Verfahrensvariante (b) vorzugsweise tertiäre Amine, wie Triethylamin und 1,4-Diazabicyclo-[2,2,2]-octan, sowie organische Zinn-Verbindungen, wie z. B. Dibutylzinndilaurat verwendet werden. Der Zusatz solcher Katalysatoren ist jedoch nicht unbedingt erforderlich.

Die Reaktionstemperatur kann bei den Verfahrensvarianten (a) und (b) innerhalb eines größeren Bereiches variiert werden. Im allgemeinen arbeitet man bei der Verfahrensvariante (a) zwischen 20 °C und 180 °C, vorzugsweise zwischen 40 °C und 120 °C und bei der Verfahrensvariante (b) zwischen 20 °C und 200 °C, vorzugsweise zwischen 60 °C und 190 °C. Die erfindungsgemäßen Verfahrensvarianten werden im allgemeinen bei Normaldruck durchgeführt.

Zur Durchführung der erfindungsgemäßen Verfahrensvarianten werden die Ausgangsstoffe gewöhnlich in etwa äquimolaren Mengen eingesetzt. Ein Überschuß der einen oder anderen Reaktionskomponenten bringt keine wesentlichen Vorteile.

Die Aufarbeitung der Reaktionsprodukte geschieht nach üblichen Methoden, z. B. durch Absaugen des ausgefallenen Produktes oder durch Herauslösen von unerwünschten Nebenprodukten aus dem Reaktionsgemisch. Zur Charakterisierung dient der Schmelzpunkt.

Die Verbindungen der allgemeinen Formel (I) eignen sich zur bekämpfung von tierischen Schädlingen, vorzugsweise Arthropoden, insbesondere Insekten, die in der Landwirtschaft, in Forsten, im Vorrats- und

Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium Punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Die erfindungsgemäßen Verbindungen der allgemeinen Formel (I) zeichnen sich durch sehr starke insektizide Wirksamkeit aus. Sie zeigen insbesondere beim Einsatz gegen Pflanzenschädigende Insekten hervorragende Wirkung, wie z. B. gegen Schmetterlingsraupen (z.B. Plutella maculipennis und Spodoptera spp.) und gegen Käferlarven (z. B. Phaedon cochleariae).

Die erfindungsgemäßen Wirkstoffe der Formel (I) eignen sich auch zur Bekämpfung von Arthropoden, die im Zusammenhang mit der Aufzucht und Haltung von Tieren z.B. von landwirtschaftlichen Nutztieren, wie z.B. Rinder, Schafe, Ziegen, Pferde, Schweine, Hühner usw., sonstigen Haustieren wie z.B. Hunde, Katzen, Stubenvögel und Aquarienfische auftreten. Durch die Bekämpfung dieser Arthropoden sollen

Ausfälle und Leistungsminderungen (bei Fleisch, Milch, Wolle, Häuten, Eiern usw.) verhindert oder verringert werden, so daß eine wirtschaftlichere und einfachere Tierhaltung möglich ist.

Die Anwendung der erfindungsgemäßen Wirkstoffe geschieht in der auf diesem Gebiet üblichen Weise, z.B. durch Tauschen oder Baden (Dippen), Sprühen (Spray), Aufgießen (Pour-on und Spot-on), Waschen, Einpudern sowie mit Hilfe von wirkstoffhaltigen Formkörpern, wie Halsbändern, Ohrmarken, Schwanzmarken, Gliedmaßenbändern, Halftern, Markierungsvorrichtungen usw. Da die Wirkstoffe im Tierkörper nicht resorbiert werden (sich also "inert" verhalten), können sie in Form von geeigneten Zubereitungen (z.B. Boli, Granulaten) auch im sogenannten "feed-through-Verfahren (z.B. über das Futter oder Trinkwasser) eingesetzt werden, wodurch die Entwicklung von Insekten im Tierkot verhindert oder verringert werden kann.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstofe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,000001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Im vorliegenden Test beziehen sich alle Prozentangaben auf Gewichtsprozente, wenn nichts anderes angegeben wird.

Herstellungsbeispiele

Beispiel 1

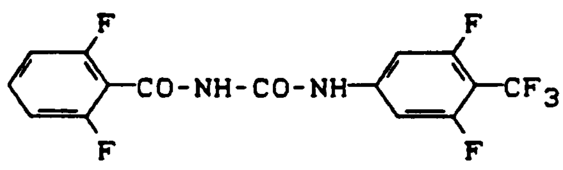

(Verfahrensvariante (a))

Zu 1,97 g (0,01 Mol) 3,5-Difluor-4-trifluormethyl-anilin in 60 ml trockenem Toluol tropft man bei 40 °C eine Lösung von 1,83 g (0,01 Mol) 2,6-Difluorbenzoylisocyanat in 10 ml trockenem Toluol. Der Ansatz wird eine Stunde bei 80 °C gerührt. Anschließend werden ca. 3/4 des Lösungsmittels unter Vakuum abdestilliert. Man fügt ca. 10 - 15 ml Petrolether hinzu und filtriert den Niederschlag ab. Er wird mit Petrolether nachgewaschen. Die Substanz wird im Vakuum bei 50 °C - 60 °C getrocknet.

Man erhält 3,6 g (95 % der Theorie) 1-(2,6-Difluor-benzoyl)-3-(3,5-difluor-4-trifluormethyl-phenyl)-harnstoff vom Schmelzpunkt 231 °C.

Analog Beispiel 1 bzw. entsprechend der allgemeinen Beschreibung der erfindungsgemäßen Verfahrensvarianten (a) und (b) können die in der nachstehenden Tabelle 2 aufgeführten Verbindungen der Formel (I) hergestellt werden.

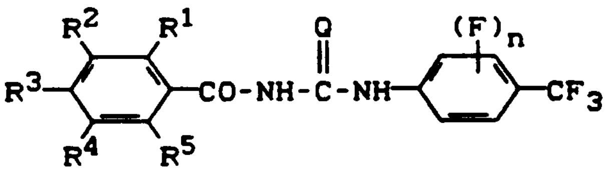

(I)

Tabelle 2: Beispiele für die Verbindungen der Formel (I)

| Bsp.-Nr. | Q | $R^1$ | $R^2$ | $R^3$ | $R^4$ | $R^5$ | 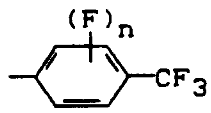 | Schmelzpunkt (°C) |
|---|---|---|---|---|---|---|---|---|
| 2 | S | Cl | H | H | H | Cl | 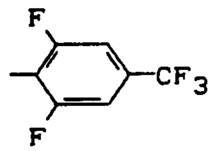 | 259 (Zers.) |
| 3 | S | Cl | H | F | H | H | 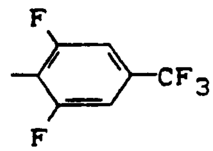 | 212 |
| 4 | S | Br | H | H | H | H | 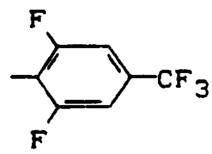 | 207 |
| 5 | S | Cl | H | H | H | H | 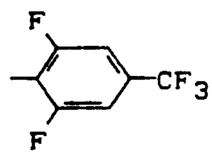 | 198 |
| 6 | O | F | H | F | H | H | 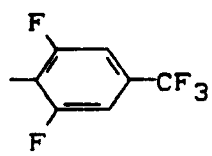 | 205 |
| 7 | O | $CH_3$ | H | H | H | H | 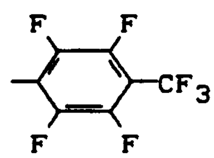 | 198 |

Tabelle 2 - Fortsetzung

| Bsp.-Nr. | Q | $R^1$ | $R^2$ | $R^3$ | $R^4$ | $R^5$ | 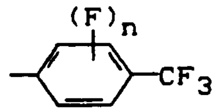 | Schmelzpunkt (°C) |
|---|---|---|---|---|---|---|---|---|
| 8 | O | F | F | F | F | F | 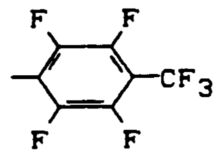 | 187 |
| 9 | O | Cl | H | Cl | F | H | 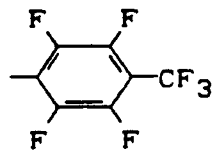 | 192 |
| 10 | O | Cl | H | H | F | H | 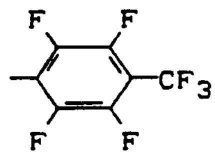 | 189 |
| 11 | O | F | H | F | H | H | 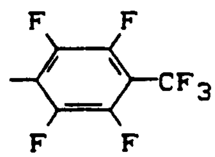 | 168 |
| 12 | S | Cl | H | H | H | H | 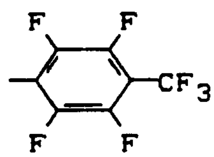 | 184 |
| 13 | S | Cl | H | H | H | F | 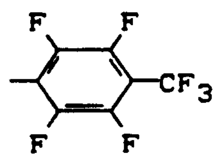 | 206 |

Tabelle 2 - Fortsetzung

| Bsp.-Nr. | Q | $R^1$ | $R^2$ | $R^3$ | $R^4$ | $R^5$ | 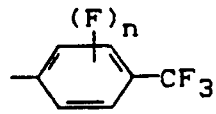 | Schmelzpunkt (°C) |
|---|---|---|---|---|---|---|---|---|
| 14 | S | F | H | H | H | F | 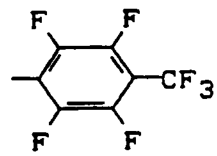 | 181 |
| 15 | O | Cl | H | H | H | H | 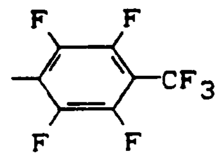 | 199 |
| 16 | O | Cl | H | F | H | H | 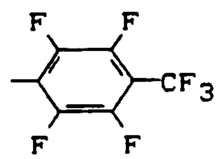 | 182 |
| 17 | O | Cl | H | H | H | F | 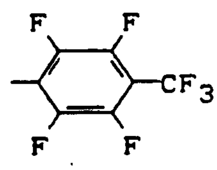 | 189 |
| 18 | O | F | H | H | H | F | 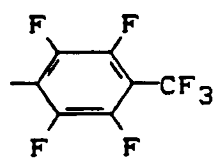 | 194 |
| 19 | S | F | H | H | H | F | 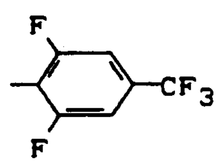 | 195 |

Tabelle 2 - Fortsetzung

| Bsp.-Nr. | Q | $R^1$ | $R^2$ | $R^3$ | $R^4$ | $R^5$ | 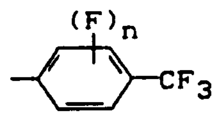 | Schmelzpunkt (°C) |
|---|---|---|---|---|---|---|---|---|
| 20 | O | Cl | H | F | H | H | 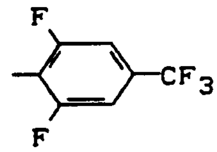 | 204 |
| 21 | O | Cl | H | H | H | H | 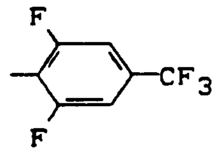 | 193 |
| 22 | O | F | H | F | H | H | 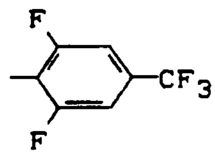 | 192 |
| 23 | O | Cl | H | H | H | F | 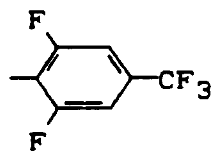 | 213 |
| 24 | O | F | H | H | H | F | 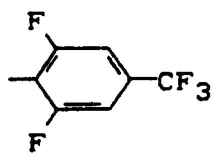 | 207 (Zers.) |
| 25 | S | Cl | H | F | H | H | 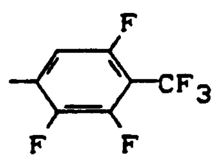 | 109 |

Tabelle 2 - Fortsetzung

| Bsp.-Nr. | Q | $R^1$ | $R^2$ | $R^3$ | $R^4$ | $R^5$ | 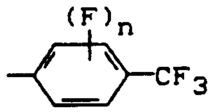 | Schmelzpunkt (°C) |
|---|---|---|---|---|---|---|---|---|
| 26 | O | Cl | H | H | H | H | 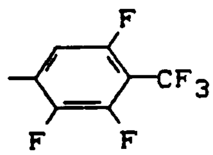 | 191 |
| 27 | O | Cl | H | F | H | H | 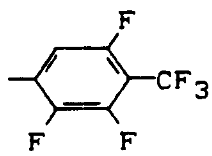 | 155 |
| 28 | O | Cl | H | H | H | F | 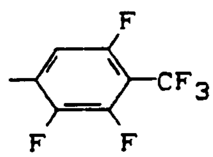 | 223 |
| 29 | O | F | H | H | H | F | 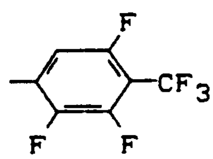 | 205 |
| 30 | O | Cl | H | H | H | H | 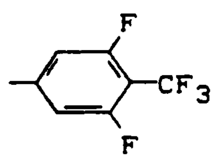 | 211 |
| 31 | O | Cl | H | F | H | H | 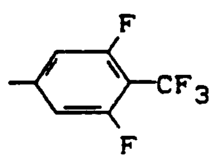 | 192 |

Tabelle 2 - Fortsetzung

| Bsp.-Nr. | Q | $R^1$ | $R^2$ | $R^3$ | $R^4$ | $R^5$ | 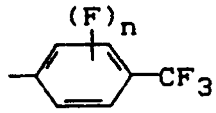 | Schmelzpunkt (°C) |
|---|---|---|---|---|---|---|---|---|
| 32 | O | Cl | H | H | H | F | 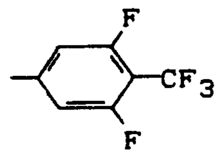 | 228 |
| 33 | S | F | H | H | H | F | 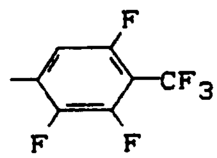 | 158 |
| 34 | S | Cl | H | H | H | F | 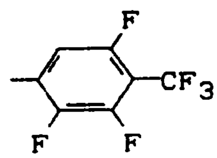 | 177 |
| 35 | S | Cl | H | H | H | H | 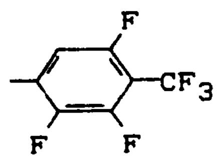 | 142 |
| 36 | S | $CH_3$ | H | H | H | H | 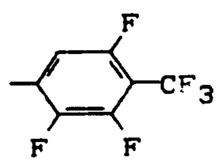 | 134 |
| 37 | S | F | H | H | H | H | 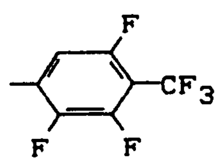 | 134 |
| 38 | S | Cl | H | H | H | Cl | 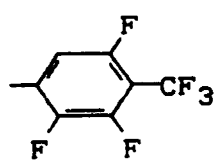 | 222 |

Tabelle 2 - Fortsetzung

| Bsp.-Nr. | Q | $R^1$ | $R^2$ | $R^3$ | $R^4$ | $R^5$ | 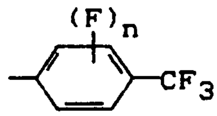 | Schmelzpunkt (°C) |
|---|---|---|---|---|---|---|---|---|
| 39 | S | Cl | H | H | F | H | 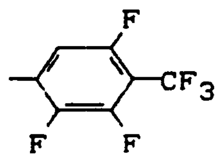 | 112 |
| 40 | S | $CF_3$ | H | H | H | H | 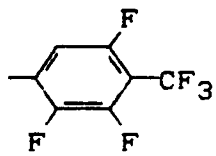 | 147 |
| 41 | S | Br | H | H | H | H | 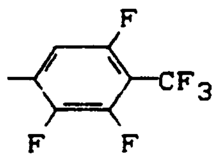 | 150 |
| 42 | S | H | H | H | H | H | 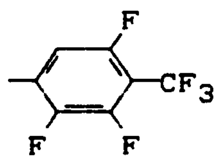 | 158 |
| 43 | O | Br | H | H | H | H | 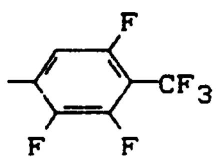 | 191 |
| 44 | O | F | H | H | H | H | 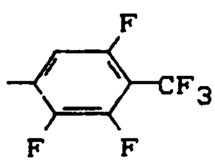 | 179 |
| 45 | O | $CH_3$ | H | H | H | H | 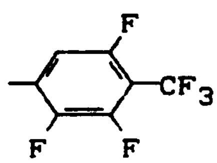 | 208 |

Tabelle 2 - Fortsetzung

| Bsp.-Nr. | Q | $R^1$ | $R^2$ | $R^3$ | $R^4$ | $R^5$ | 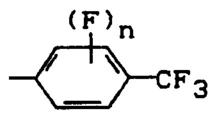 | Schmelzpunkt (°C) |
|---|---|---|---|---|---|---|---|---|
| 46 | O | Cl | H | H | F | H | 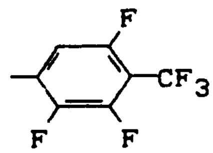 | 177 |
| 47 | O | H | H | H | H | H | 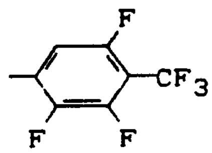 | 245 |
| 48 | O | F | H | F | H | H | 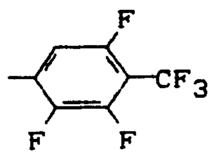 | 190 |
| 49 | O | Cl | H | Cl | F | H | 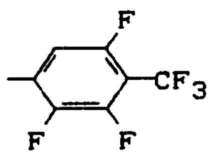 | 177 |
| 50 | O | F | F | F | F | F | 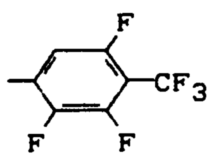 | 166 |
| 51 | O | $NO_2$ | H | H | H | H | 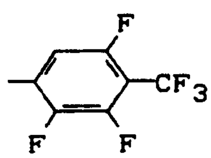 | 189 |
| 52 | O | Cl | H | H | H | Cl | 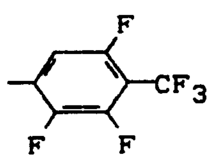 | 231 |

Tabelle 2 - Fortsetzung

| Bsp.-Nr. | Q | $R^1$ | $R^2$ | $R^3$ | $R^4$ | $R^5$ | 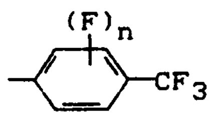 | Schmelzpunkt (°C) |
|---|---|---|---|---|---|---|---|---|
| 53 | O | Cl | H | Cl | H | H | 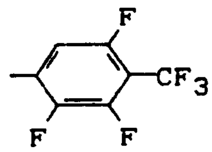 | 173 |
| 54 | S | F | H | H | H | F | 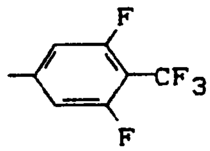 | 201 |
| 55 | S | Cl | H | H | H | F | 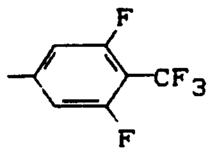 | 212 |
| 56 | S | Cl | H | F | H | H | 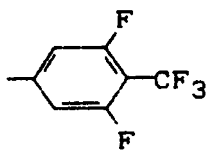 | 123 |
| 57 | S | Cl | H | H | H | H | 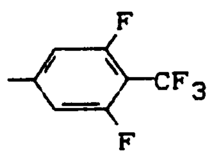 | 159 |
| 58 | S | Cl | H | H | H | Cl | 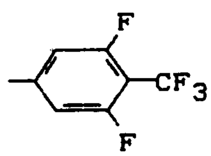 | 228 |
| 59 | S | F | H | H | H | H | 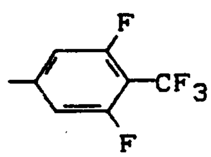 | 123 |

Tabelle 2 - Fortsetzung

| Bsp.-Nr. | Q | $R^1$ | $R^2$ | $R^3$ | $R^4$ | $R^5$ | 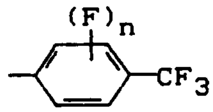 | Schmelzpunkt (°C) |
|---|---|---|---|---|---|---|---|---|
| 60 | S | $CH_3$ | H | H | H | H | 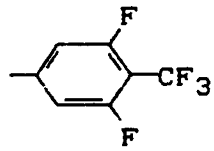 | 143 |
| 61 | S | H | H | H | H | H | 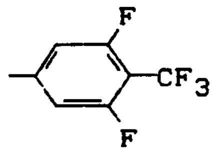 | 141 |
| 62 | O | F | H | F | H | H | 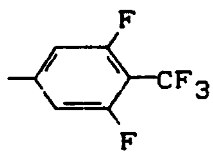 | 199 |
| 63 | O | Cl | H | H | F | H | 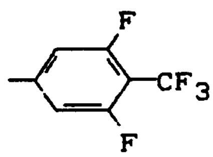 | 191 |
| 64 | S | $CF_3$ | H | H | H | H | 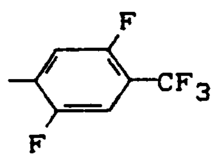 | 145 |
| 65 | S | Cl | H | H | F | H | 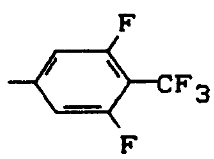 | 153 |
| 66 | O | H | H | H | H | H | 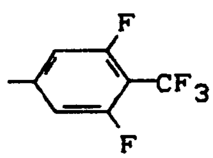 | 234 |

Tabelle 2 - Fortsetzung

| Bsp.-Nr. | Q | $R^1$ | $R^2$ | $R^3$ | $R^4$ | $R^5$ | 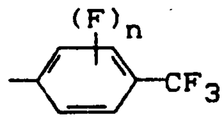 | Schmelzpunkt (°C) |
|---|---|---|---|---|---|---|---|---|
| 67 | O | Br | H | H | H | H | 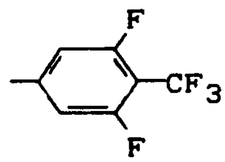 | 215 |
| 68 | O | $CH_3$ | H | H | H | H | 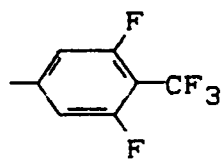 | 230 |
| 69 | O | F | F | F | F | F | 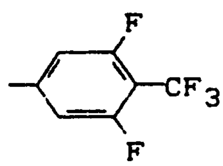 | 176 |
| 70 | O | F | H | H | H | F | 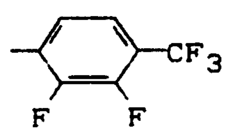 | 195 |
| 71 | O | Cl | H | H | H | F | 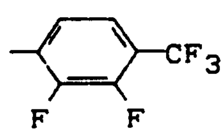 | 198 |
| 72 | S | F | H | H | H | F | 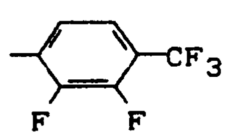 | 193 |
| 73 | S | Cl | H | F | H | H | 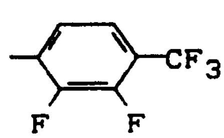 | 148 |

Tabelle 2 - Fortsetzung

| Bsp.-Nr. | Q | $R^1$ | $R^2$ | $R^3$ | $R^4$ | $R^5$ | 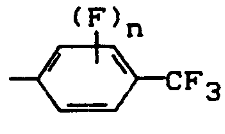 | Schmelzpunkt (°C) |
|---|---|---|---|---|---|---|---|---|
| 74 | S | Cl | H | H | H | F | 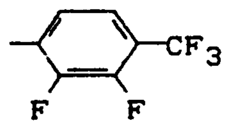 | 223 |

Ausgangsstoffe der Formel (II)

Beispiel (II-1)

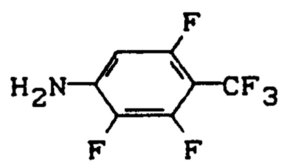

In einem Autoklaven von 0,7 l Inhalt aus VA (VA = Edelstahl) werden 110 g 2,3,4,6-Tetrafluorbenzotrifluorid in 200 ml Tetrahydrofuran vorgelegt und 30 ml flüssiger Ammoniak aufgedrückt. Anschließend wird für 3 Stunden auf 100 °C erhitzt (max. Druck: 10 bar). Nach Abkühlen wird entspannt und die flüssige Phase einer fraktionierten Destillation unterworfen. Zuerst werden bei einem Siedepunkt von 47 °C -48 °C/8 mbar 42 g 2,3,5-Trifluor-6-trifluormethylanilin erhalten, dann nach einem Zwischenlauf bei einem Siedepunkt von 75 °C -78 °C/8 mbar 46 g 2,3,5-Trifluor-4-trifluormethylanilin.

Beispiel (II-2)

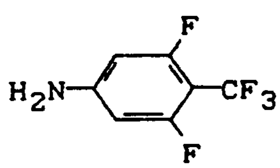

In einem VA-Autoklaven werden 200 ml Tetrahydrofuran und 50 g 2,4,6-Trifluorbenzotrifluorid vorgelegt und 30 ml flüssiger Ammoniak aufgedrückt. Anschließend wird unter Rühren für 6 Stunden auf 120 °C erhitzt. Nach Abkühlen und Entspannen wird die Reaktionsmischung einer fraktionierten Destillation unterworfen.
Bei einem Siedepunkt von 57 °C - 58 °C/12 mbar werden 15 g 2-Trifluormethyl-3,5-difluoranilin erhalten. Nach einem kleinen Zwischenlauf gehen bei einem Siedepunkt von 103 °C - 105 °C/16 mbar 32 g 3,5-Difluor-4-trifluormethylanilin über (Schmelzpunkt: 66 °C).

Beispiel (II-3)

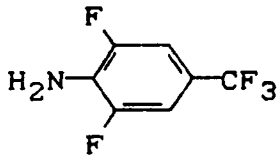

In einem VA-Autoklaven werden 500 g 3,4,5-Trifluor-benzotrifluorid vorgelegt, 2000 ml Tetrahydrofuran zugegeben und 150 g flüssigen Ammoniak aufgedrückt. Unter Rühren wird der Autoklav für 5 Stunden auf 120 °C - 130 °C erhitzt, dann abgekühlt und bei 20 °C entspannt. Durch Destillation werden neben dem Lösungsmittel und Ausgangsmaterial 272 g 2,6-Difluor-4-trifluormethylanilin (Siedepunkt 58 °C - 60 °C/16 mbar) erhalten.

Beispiel (II-4)

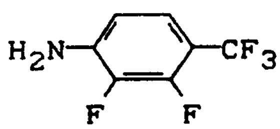

In einem Autoklaven werden 200 g 2,3,4-Trifluor-benzotrifluorid in 500 ml Tetrahydrofuran vorgelegt und 60 ml flüssiger Ammoniak aufgedrück. Nach 6 Stunden Rühren bei 130 °C (max. Druck 18 bar) wird abgekühlt und entspannt. Durch Destillation werden bei einem Siedepunkt von 92 °C - 93 °C/26 mbar 92 g (2,3-Difluor-4-trifluormethylanilin erhalten.

Beispiel A

Phaedon-Larven-Test

Lösungsmittel: 15 Gewichtsteile Dimethylformamid
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigten z. B. die Verbindungen der Herstellungsbeispiele (1), (30) und (32) bei einer beispielhaften Konzentration von 0,0001 % nach 10 Tagen eine 100 %ige Abtötung.

Beispiel B

Plutella-Test

Lösungsmittel: 15 Gewichtsteile Dimethylformamid
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter

noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z. B. die Verbindungen der Herstellungsbeispiele (1), (26), (30) und (31) bei einer beispielhaften Konzentration von 0,00001 % nach 7 Tagen eine Abtötung von 100 %.

Beispiel C

Spodoptera-Test

Lösungsmittel: 15 Gewichtsteile Dimethylformamid
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z. B. die Verbindungen der Herstellungsbeispiele (1), (25), (26), (27), (28), (29), (30), (31) und (32) bei einer beispielhaften Konzentration von 0,0001 % nach 7 Tagen eine Abtötung von 100 %.

Beispiel D

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigten z. B. die Verbindungen der Herstellungsbeispiele (25), (26), (27) und (29) bei einer beispielhaften Konzentration von 100 ppm einen Abtötungsgrad von 100 %.

## Patentansprüche

1. Substituierte Benzoyl(thio)harnstoffe der allgemeinen Formel (I),

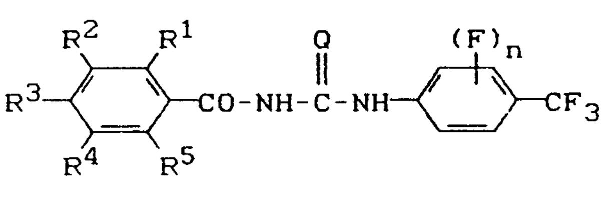

(I)

in welcher

- Q für Sauerstoff oder Schwefel steht,
- $R^1$ für Wasserstoff, Halogen, Nitro, Methyl oder Trifluormethyl steht,
- $R^2$ für Wasserstoff, Fluor oder Chlor steht,
- $R^3$ für Wasserstoff, Fluor oder Chlor steht,
- $R^4$ für Wasserstoff oder Fluor steht,
- $R^5$ für Wasserstoff oder Halogen steht und
- n für die Zahlen 2, 3 oder 4 steht,

ausgenommen die Verbindungen, in welchen zugleich

$R^1$ und $R^5$ für Fluor stehen und
$R^2$ und $R^4$ für Wasserstoff stehen und
$R^3$ für Wasserstoff oder Fluor steht und
Q für Sauerstoff steht und
$(F)_n$ für 2,5-Difluor steht.

**2.** Verbindungen gemäß Anspruch 1, wobei

Q für Sauerstoff oder Schwefel steht,
$R^1$ für Wasserstoff, Fluor, Chlor, Nitro, Methyl oder Trifluormethyl steht,
$R^2$ für Wasserstoff, Fluor oder Chlor steht,
$R^3$ für Wasserstoff, Fluor oder Chlor steht,
$R^4$ für Wasserstoff oder Fluor steht,
$R^5$ für Wasserstoff, Fluor oder Chlor steht und
n für die Zahlen 2, 3 oder 4 steht.

**3.** Verbindungen gemäß Anspruch 1, wobei

Q für Sauerstoff oder Schwefel steht,
$R^1$ für Fluor oder Chlor steht,
$R^2$ für Wasserstoff steht,
$R^3$ für Wasserstoff oder Fluor steht,
$R^4$ für Wasserstoff oder Fluor steht,
$R^5$ für Wasserstoff oder Fluor steht und
n für die Zahlen 2, 3 oder 4 steht.

**4.** Verbindung der Formel

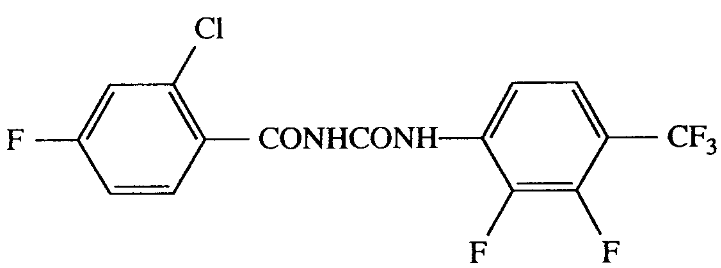

**5.** Verfahren zur Herstellung der neuen substituierten Benzoyl(thio)harnstoffe gemäß Anspruch 1, dadurch gekennzeichnet, daß man

(a) substituierte Aniline der allgemeinen Formel (II),

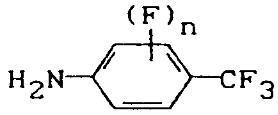

(II)

in welcher

n die in Anspruch 1 angegebene Bedeutung hat,

mit Benzoyliso(thio)cyanaten der allgemeinen Formel (III)

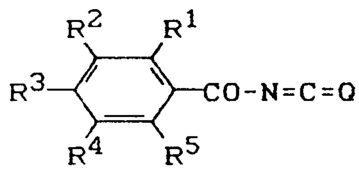 (III)

in welcher

Q, $R^1$, $R^2$, $R^3$, $R^4$ und $R^5$ die in Anspruch 1 angegebenen Bedeutungen haben,

gegebenenfalls in Gegenwart eines Verdünnungsmittels umsetzt, oder

(b) substituierte Phenyliso(thio)cyanate der allgemeinen Formel (IV)

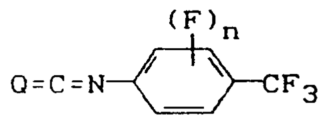 (IV)

in welcher

Q und n die in Anspruch 1 angegebenen Bedeutungen haben,

mit Benzoesäureamiden der allgemeinen Formel (V)

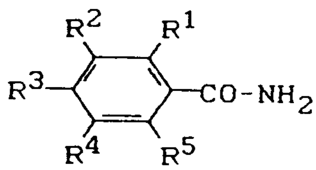 (V)

in welcher

$R^1$, $R^2$, $R^3$, $R^4$ und $R^5$ die in Anspruch 1 angegebenen Bedeutungen haben,

gegebenenfalls in Gegenwart eines Katalysators und gegebenenfalls in Gegenwart eines Verdünnungsmittels umsetzt.

**6.** Schädlingsbekämpfungsmittel, gekennzeichnet durch einen Gehalt an mindestens einer Verbindung gemäß den Ansprüchen 1 bis 4.

**7.** Verwendung von Verbindungen gemäß den Ansprüchen 1 bis 4 zur Bekämpfung von Schädlingen.

**8.** Verfahren zur Bekämpfung von Schädlingen, dadurch gekennzeichnet, daß man Verbindungen gemäß den Ansprüchen 1 bis 4 auf Schädlinge und/oder ihren Lebensraum einwirken läßt.

**9.** Verfahren zur Herstellung von Schädlingsbekämpfungsmitteln, dadurch gekennzeichnet, daß man Verbindungen gemäß den Ansprüchen 1 bis 4 mit Streckmitteln und/oder oberflächenaktiven Mitteln vermischt.

**10.** Substituierte Phenyliso(thio)cyanate der allgemeinen Formel (IV)

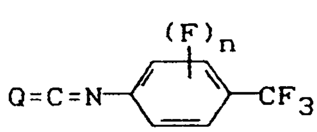 (IV)

in welcher

Q für Sauerstoff oder Schwefel steht und
n für die Zahlen 2, 3 oder 4 steht,
ausgenommen die Verbindungen, in welchen zugleich
$(F)_n$ für 2,5-Difluor steht und
Q für Sauerstoff steht.

## Claims

**1.** Substituted benzoyl(thio)ureas of the general formula (I)

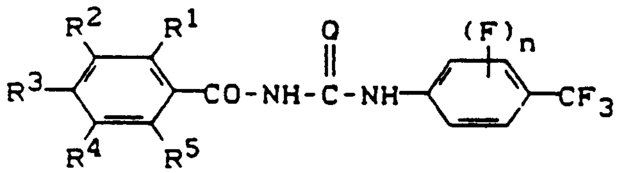

(I)

in which
Q represents oxygen or sulphur,
$R^1$ represents hydrogen, halogen, nitro, methyl or trifluoromethyl,
$R^2$ represents hydrogen, fluorine or chlorine,
$R^3$ represents hydrogen, fluorine or chlorine,
$R^4$ represents hydrogen or fluorine,
$R^5$ represents hydrogen or halogen and
n represents the numbers 2, 3 or 4,
with the exception of the compounds in which at the same time
$R^1$ and $R^5$ represent fluorine and
$R^2$ and $R^4$ represent hydrogen and
$R^3$ represents hydrogen or fluorine and
Q represents oxygen and
$(F)_n$ represents 2,5-difluoro.

**2.** Compounds according to Claim 1, wherein
Q represents oxygen or sulphur,
$R^1$ represents hydrogen, fluorine, chlorine, nitro, methyl or trifluoromethyl,
$R^2$ represents hydrogen, fluorine or chlorine,
$R^3$ represents hydrogen, fluorine or chlorine,
$R^4$ represents hydrogen or fluorine,
$R^5$ represents hydrogen, fluorine or chlorine and
n represents the numbers 2, 3 or 4.

**3.** Compounds according to Claim 1, wherein
Q represents oxygen or sulphur,
$R^1$ represents fluorine or chlorine,
$R^2$ represents hydrogen,
$R^3$ represents hydrogen or fluorine,
$R^4$ represents hydrogen or fluorine,
$R^5$ represents hydrogen or fluorine and
n represents the numbers 2, 3 or 4.

**4.** Compound of the formula

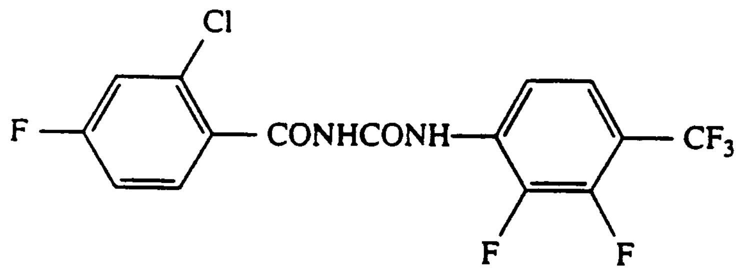

**5.** Process for the preparation of the new substituted benzoyl(thio)ureas according to Claim 1, characterised in that

(a) substituted anilines of the general formula (II)

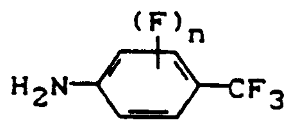

(II)

in which

n  has the meaning given in Claim 1,

are reacted with benzoyl iso(thio)cyanates of the general formula (III)

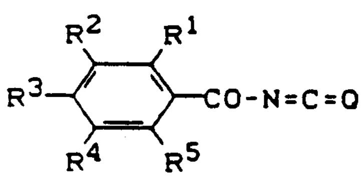

(III)

in which

Q, $R^1$, $R^2$, $R^3$, $R^4$ and $R^5$  have the meanings given in Claim 1,

if appropriate in the presence of a diluent, or

(b) substituted phenyl iso(thio)cyanates of the general formula (IV)

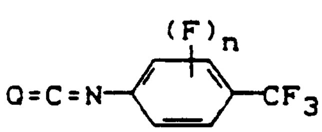

(IV)

in which

Q and n  have the meanings given in Claim 1,

are reacted with benzamides of the general formula (V)

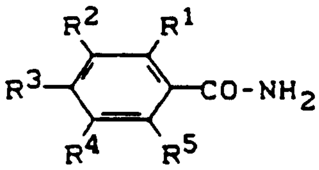

(V)

in which
$R^1$, $R^2$, $R^3$, $R^4$ and $R^5$ have the meanings given in Claim 1,
if appropriate in the presence of a catalyst and if appropriate in the presence of a diluent.

6. Pesticide, characterised in that it contains at least one compound according to Claims 1 to 4.

7. Use of compounds according to Claims 1 to 4 for the control of pests.

8. Method of combating pests, characterised in that compounds according to Claims 1 to 4 are allowed to act on pests and/or their habitat.

9. Process for the preparation of pesticides, characterised in that compounds according to Claims 1 to 4 are mixed with extenders and/or surface-active agents.

10. Substituted phenyl iso(thio)cyanates of the general formula (IV)

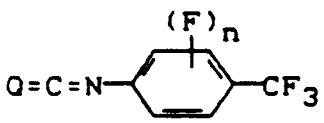

(IV)

in which
Q represents oxygen or sulphur and
n represents the numbers 2, 3 or 4,
with the exception of the compounds in which at the same time
$(F)_n$ represents 2,5-difluoro and
Q represents oxygen.

**Revendications**

1. Benzoyl(thio)urées substituées de formule générale (I),

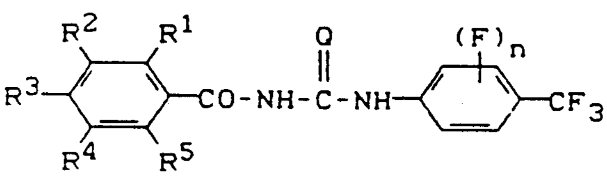

(I)

dans laquelle
Q représente l'oxygène ou le soufre,
$R^1$ représente l'hydrogène, un halogène, un groupe nitro, méthyle ou trifluorométhyle,
$R^2$ est l'hydrogène, le fluor ou le chlore,
$R^3$ est l'hydrogène, le fluor ou le chlore,
$R^4$ est l'hydrogène ou le fluor,
$R^5$ est l'hydrogène ou un halogène et
n représente les nombres 2, 3 ou 4,
à l'exception des composés dans lesquels, simultanément

| | |
|---|---|
| $R^1$ et $R^5$ | représentent du fluor et |
| $R^2$ et $R^4$ | sont de l'hydrogène et |
| $R^3$ | est l'hydrogène ou le fluor et |
| Q | est l'oxygène et |
| $(F)_n$ | est un groupe 2,5-difluoro. |

**2.** Composés suivant la revendication 1, dans lesquels

| | |
|---|---|
| Q | est l'oxygène ou le soufre, |
| $R^1$ | est l'hydrogène, le fluor, le chlore, un groupe nitro, méthyle ou trifluorométhyle, |
| $R^2$ | est l'hydrogène, le fluor ou le chlore, |
| $R^3$ | est l'hydrogène, le fluor ou le chlore, |
| $R^4$ | est l'hydrogène ou le fluor, |
| $R^5$ | est l'hydrogène, le fluor ou le chlore et |
| n | a les valeurs 2, 3 ou 4. |

**3.** Composés suivant la revendication 1, dans lesquels

| | |
|---|---|
| Q | est l'oxygène ou le soufre, |
| $R^1$ | est le fluor ou le chlore, |
| $R^2$ | est l'hydrogène, |
| $R^3$ | est l'hydrogène ou le fluor, |
| $R^4$ | est l'hydrogène ou le fluor, |
| $R^5$ | est l'hydrogène ou le fluor et |
| n | a les valeurs 2, 3 ou 4. |

**4.** Le composé de formule

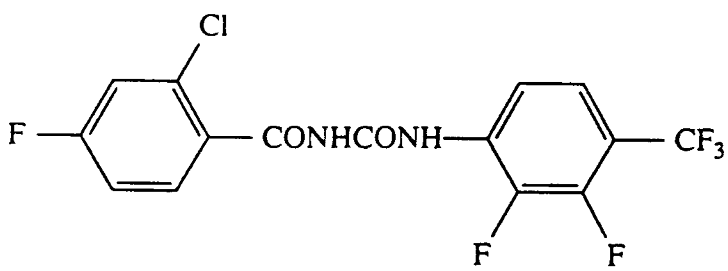

**5.** Procédé de production des benzoyl(thio)urées substituées nouvelles suivant la revendication 1, caractérisé en ce que :

(a) on fait réagir éventuellement en présence d'un diluant, des anilines substituées de formule générale (II),

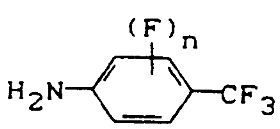

(II)

dans laquelle

n a la définition indiquée dans la revendication 1,

avec des benzoyliso(thio)cyanates de formule générale (III)

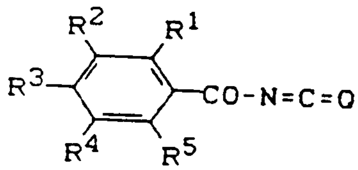

(III)

dans laquelle

Q, $R^1$, $R^2$, $R^3$, $R^4$ et $R^5$ ont les définitions indiquées dans la revendication 1,

éventuellement en présence d'un diluant, ou bien

(b) on fait réagir des phényliso(thio)cyanates substitués de formule générale (IV)

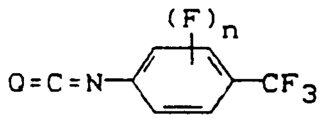

(IV)

dans laquelle

Q et n ont les définitions indiquées dans la revendication 1,

avec des amides d'acide benzoïque de formule générale (V)

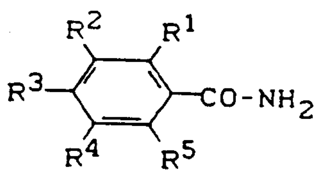

(V)

dans laquelle

$R^1$, $R^2$, $R^3$, $R^4$ et $R^5$ ont les définitions indiquées dans la revendication 1,

le cas échéant en présence d'un catalyseur et en la présence éventuelle d'un diluant.

6. Compositions pesticides, caractérisées par une teneur en au moins un composé suivant les revendications 1 à 4.

7. Utilisation de composés suivant les revedications 1 à 4 pour combattre des parasites.

8. Procédé pour combattre des parasites, caractérisé en ce qu'on fait agir sur des parasites et/ou sur leur habitat des composés suivant les revendications 1 à 4.

9. Procédé de préparation de compositions pesticides, caractérisé en ce qu'on mélange des composés suivant les revendications 1 à 4 avec des diluants et/ou des agents tensio-actifs.

10. Phényliso(thio)cyanates substitués de formule générale (IV)

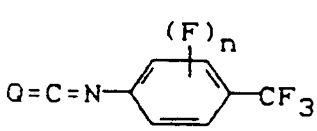

(IV)

dans laquelle

Q représente l'oxygène ou le soufre et

n a les valeurs 2, 3 ou 4,

excepté les composés dans lesquels, simultanément

$(F)_n$ est un groupe 2,5-difluoro et
Q est l'oxygène.